# EUROPÄISCHE PATENTANMELDUNG

(11) **EP 4 592 529 A1**
(43) Veröffentlichungstag der Anmeldung: **30.07.2025**
(21) Anmeldenummer: 25154061.3
(22) Anmeldetag: 27.01.2025
(51) Int. Cl.: F04B 43/12, A61M 5/142, A61M 39/28, F04B 53/16, F04B 53/22

(54) **MEDIZINISCHE SCHLAUCHPUMPE**

(30) Priorität: 29.01.2024 DE 102024102479
(71) Anmelder: B. Braun Melsungen AG, 34212 Melsungen (DE)
(72) Erfinder: RICHARDT, Mario, 34289 Zierenberg (DE); SCHWENDICH, Wilhelm, 36100 Petersberg (DE); SCHWALM, Matthias, 34613 Schwalmstadt (DE)
(74) Vertreter: Winter, Brandl - Partnerschaft mbB

(57) **Zusammenfassung**

Offenbart ist eine medizinische Schlauchpumpe (1), insbesondere zur medizinischen Dosierung, mit einer Schlauchaufnahme, die ausgestaltet ist, dass in sie ein Schlauch (14) einlegbar und festlegbar ist, und mit einem Klemmmechanismus (20) mit einem ersten Klemmbacken (22, 22'), der gegen einen zweiten Klemmbacken (23) in eine Klemmstellung vorgespannt ist, in welcher der Schlauch (14) klemmbar ist, insbesondere geklemmt ist, wobei der erste Klemmbacken (22, 22') aus der Klemmstellung in eine Offenstellung betätigbar ist, in welcher der Schlauch (14) freigebbar, insbesondere freigegeben, und/oder einlegbar ist, und mit einem Sperrhebel (28), der von wenigstens einem Federelement (36) mittelbar oder unmittelbar gegen den ersten Klemmbacken (22, 22') spannbar ist, insbesondere gespannt ist, sodass der erste Klemmbacken (22, 22') in der Offenstellung mittelbar oder unmittelbar am Sperrhebel (28) abgestützt ist.

## Beschreibung

### Technisches Gebiet

Die vorliegende Offenbarung betrifft eine medizinische Schlauchpumpe.

Die Dosierung medizinischer Wirkstoffe oder die Förderung von Blut in der extrakorporalen Blutbehandlung kann über eine Schlauchpumpe erfolgen. Ein mögliches Wirkprinzip einer solchen Schlauchpumpe ist, einem Schlauch, durch welchen ein Fluid geleitet wird, mittels einer Verdrängereinheit der Schlauchpumpe eine peristaltische Bewegung aufzuprägen, wodurch das Fluid von einem Wirkstoffbehälter hin zu einem Zugang am Patienten gefördert wird.

### Stand der Technik

Eine Schlauchpumpe radialer Bauart ist im Patent EP 3 061 473 B1 der Anmelderin offenbart. Die gezeigte Schlauchpumpe zur extrakorporalen Blutbehandlung hat einen motorischen Drehantrieb mit einem Rotor, an dem radial außen Quetschelemente vorgesehen sind, die bei Rotation wiederkehrend an einem gehäusefest und bogenförmig abgestützten Schlauchabschnitt in einer Schlauchlängsrichtung abgleiten oder abrollen.

Eine Schlauchpumpe linearer Bauart ist im Patent EP 0 484 717 B1 der Anmelderin gezeigt. Sie hat eine Schlauchaufnahme für einen Schlauch, durch welchen Fluid gefördert wird, sowie eine Verdrängereinheit mit einer Vielzahl schieberartiger Verdränger, die entlang der Schlauchaufnahme nebeneinander angeordnet sind und quer zu der Aufnahme linearbeweglich in einem schachtartigen Gehäuseabschnitt geführt sind. Der Schlauch wird in die Schlauchaufnahme eingesetzt und eine Gehäuseklappe wird geschlossen, sodass der Schlauch von der Gehäuseklappe in der Schlauchaufnahme festgelegt ist. Bei geschlossener Gehäuseklappe arbeiten die zeitversetzt betätigten Verdränger gegen den Schlauch, was zu dessen Quetschung an der Gehäuseklappe und zu einer Bewegung des Schlauches nach peristaltischem Muster führt, was wiederum die Förderung des Fluids zur Folge hat.

Um sicherzustellen, dass ein Fluidfluss zum Patienten nur bei Bedarf, das heißt nur im Betrieb der Schlauchpumpe, und nicht beispielsweise bereits beim Einlegen des Schlauches in die Schlauchpumpe, erfolgt, ist ein redundantes Klemmensystem üblich. Zum einen ist hierzu an dem Schlauch selbst eine Schlauchklemme vorgesehen, zum anderen verfügt die Schlauchpumpe über einen eigenen Klemmenmechanismus, dessen Klemme mit dem Einlegen des Schlauchs und Schließen der Gehäuseklappe aktiviert wird.

In solcher Bauart sind aus dem Sortiment der Anmelderin Schlauchpumpen unter der Bezeichnung Infusomat ^{®} space und Infusomat ^{®} space^{plus} bekannt, deren Klemme einen festen und einen beweglichen Klemmbacken hat. Der bewegliche Klemmbacken ist mit einem von einem Anwender tastbaren Druckhebel gekoppelt, über den er in eine Offenstellung betätigt werden kann, in welcher die Klemme offensteht und der Schlauch eingelegt, bzw. entnommen werden kann. In dieser Offenstellung ist der bewegliche Klemmbacken mit einem Sperrhebel des Klemmenmechanismus verrastet und festgelegt. Der Sperrhebel ist wiederum von einem als Spiralfeder ausgestalteten Federelement mittelbar gegen den beweglichen Klemmbacken gespannt, sodass die Verrastung und somit die Offenstellung der Klemme gesichert ist.

Der Sperrhebel und die Spiralfeder weisen eine geringe Größe im Millimeterbereich auf, sodass ihre Montage diffizil und fehleranfällig ist. Zudem führt der Sperrhebel eine Rotationsbewegung aus, wohingegen die als Druckfeder gestaltete Spiralfeder eher für lineare Bewegungen ausgelegt ist.

### Zusammenfassung der Offenbarung

Die Aufgabe der vorliegenden Offenbarung ist es, eine medizinische Schlauchpumpe zu schaffen, die die Nachteile des Stands der Technik reduziert oder ausräumt und die insbesondere gegenüber dem Stand der Technik vorrichtungstechnisch vereinfacht ist.

Die Aufgabe wird gelöst durch eine medizinische Schlauchpumpe, insbesondere zur medizinischen Dosierung, insbesondere zur Dosierung von Wirkstoffen oder zur Ernährung, oder zur Förderung von Blut, insbesondere in einem extrakorporalen Blutkreislauf. Die Schlauchpumpe kann insbesondere eine Komponente einer Dialysemaschine sein oder eine Infusionspumpe oder eine Spritzenpumpe. Die Schlauchpumpe hat offenbarungsgemäß eine Schlauchaufnahme, die vorgesehen und ausgestaltet ist, dass in sie ein Schlauch, insbesondere ein Infusionsschlauch, einlegbar ist und festlegbar ist. Die offenbarungsgemäße Schlauchpumpe hat zudem einen Klemmenmechanismus mit einem ersten Klemmbacken, der gegen einen zweiten Klemmbacken in eine Klemmstellung vorgespannt ist, in welcher ein eingelegter Schlauch klemmbar, insbesondere geklemmt, ist. Aus der Klemmstellung heraus ist der erste Klemmbacken in seine Offenstellung betätigbar, in welcher der Schlauch freigebbar, insbesondere freigegeben, und/oder einlegbar ist. Des Weiteren hat der Klemmenmechanismus einen Sperrhebel, der von wenigstens einem Federelement mittelbar oder unmittelbar gegen den ersten Klemmbacken spannbar, insbesondere gespannt ist, sodass der erste Klemmbacken in der Offenstellung mittelbar oder unmittelbar am Sperrhebel abgestützt ist, insbesondere derart, dass er in der Offenstellung gesperrt ist. Offenbarungsgemäß ist der Sperrhebel mit dem Federelement einstückig, insbesondere stoffeinstückig, gebildet.

Die einstückige Ausführung hat den Vorteil, dass nicht mehr zwei kleine Bauteile aufeinander abgestimmt, positioniert und montiert werden müssen, sondern die Teileanzahl ist von zwei auf eins reduziert. Das vereinfacht die Montage erheblich und macht sie weniger fehleranfällig.

In einer möglichen Ausgestaltung ist die Schlauchaufnahme von einem Schlauchfach und einem das Schlauchfach verschließenden Schlauchfachdeckel gebildet. In das Schlauchfach ist der Schlauch bei geöffnetem Schlauchfachdeckel einlegbar und durch das Schließen Schlauchfachdeckels festlegbar, insbesondere festgelegt.

Gemäß einer bevorzugten Weiterbildung ist das Federlement des Sperrhebels als Federlasche ausgebildet und insbesondere an einen Grundkörper des Sperrhebels angeformt.

Da von medizinischen Schlauchpumpen ein hohes Maß an Verfügbarkeit und Zuverlässigkeit gefordert wird und das sichere Einlegen und Klemmen, sowie Freigeben eines Schlauches eine Grundvoraussetzung für den Betrieb sind, ist der einstückig aus Grundkörper und Federlasche gebildete Sperrhebel vorzugsweise auf mehrere zehntausend Betätigungen ausgelegt.

Grundlegend erweist sich gehäuseseitig eine rotierende Lagerung des Grundkörpers als vorteilhaft. Eine Federwirkung wird auf einfache Weise erreicht, indem gemäß einer Weiterbildung in der Rotationsebene die Federlasche gegen den Grundkörper abgewinkelt ist. Beide, die Federlasche und der Grundkörper, bilden auf diese Weise eine Art V-Form in der Rotationsebene.

Gemäß einer bevorzugten Weiterbildung ist die Federlasche einerseits gehäuseseitig abgestützt und andererseits spannt sie den Grundkörper des Sperrhebels mittelbar - insbesondere über einen von einem Anwender tastbaren Druckhebel, mit dem der bewegliche Klemmbacken gekoppelt ist und über den der bewegliche Klemmbacken in eine Offenstellung betätigt/ getastet werden kann - oder unmittelbar gegen den beweglichen Klemmbacken.

Eine besonders robuste und einfache Form hat die Federlasche gemäß einer Weiterbildung, in der sie geschwungen, insbesondere S-förmig geschwungen, ausgebildet, insbesondere am Grundkörper angebunden, ist.

Die S-förmige Ausbildung/ Anbindung stellt eine Kraftübertragung und Dauerfestigkeit während Betätigungen des beweglichen Klemmbackens in die Offenstellung und Klemmstellung sicher und stellt eine kraftflussoptimierte Form der Federlasche dar.

Besonders vorteilhaft bezüglich der Krafteinleitung von der Federlasche in den Grundkörper ist es, wenn gemäß einer Weiterbildung eine Richtung, mit der die Federlasche an den Grundkörper angebunden, insbesondere angeformt ist, eine Rotationsachse des Sperrhebels schneidet.

Vorzugsweise ist die Federlasche etwa oder genau in einem rechten Winkel angebunden, insbesondere angeformt.

Die oben genannten V-Form kann einen Öffnungswinkel von kleiner als 90° aufweisen. Der Öffnungswinkel ist insbesondere mit Bezug auf die Rotationsachse des Sperrhebels zwischen einem Endabschnitt der Federlasche, mit welchem die Federlasche gehäuseseitig abstützbar/ abgestützt ist, und einem Endabschnitt des Grundkörpers, mit welchem der Grundkörper mittelbar oder unmittelbar gegen den beweglichen Klemmbacken spannbar/ gespannt ist, bemessen.

Insbesondere ist der Endabschnitt der im rechten Winkel angebundenen, insbesondere angeformten, Federlasche mit einem Öffnungswinkel kleiner als 90° gegen den Grundkörper abgewinkelt.

Gemäß einer Weiterbildung ist ein Endabschnitt der Federlasche mit einem Linienkontakt, der sich vorzugsweise als Normale zur Rotationsebene erstreckt, oder mit einem Flächenkontakt gehäuseseitig abgestützt. Der Linienkontakt ermöglicht dabei ein gutes Gleiten des Endabschnitts der Federlasche am gehäuseseitigen Bauteil oder einer gehäuseseitigen Abstützfläche und bietet dennoch einen guten Schutz gegen Verschleiß.

Gemäß einer Weiterbildung, insbesondere um den zuletzt genannten Linienkontakt zu realisieren, hat ein Endabschnitt, mit dem die Federlasche gehäuseseitig abgestützt ist, eine zur gehäuseseitigen Abstützfläche hin konvex gekrümmte, insbesondere teilzylindrische, Außenmantelfläche.

Alternativ oder ergänzend ist der Endabschnitt, mit dem die Federlasche gehäuseseitig abgestützt ist, gebogen. Der gebogene Endabschnitt ermöglicht ein freies Gleiten auf einer gehäuseseitigen Anlage- oder Abstützfläche. Hierzu ist insbesondere keine weitere formgebundene Führung notwendig.

Gemäß einer Weiterbildung ist eine Breite der Federlasche so ausgelegt ist, dass ein Wegkippen der Federlasche aus der Rotationsebene hinaus vermieden ist oder zumindest minimiert ist.

Vorzugsweise ist eine Breite der Federlasche, insbesondere bemessen in einer Richtung senkrecht zur Rotationsebene, wenigstens ein 2-faches bis etwa ein 10-faches einer Dicke der Federlasche, insbesondere bemessen in der Rotationsebene.

Gemäß einer bevorzugten Weiterbildung weist der Grundkörper einen, vorzugsweise zylindrischen, Lagerabschnitt auf, der gehäuseseitig rotierbar gelagert ist. An den Lagerabschnitt schließt sich, vorzugsweise in Richtung der Rotationsache, ein zum Lagerabschnitt verjüngter Anbindungsabschnitt an, an dem die Federlasche angebunden ist.

In anderer Ausgestaltung sind beide, der Lagerabschnitt und der Anbindungsabschnitt, zylindrisch, und insbesondere von gleichen Durchmesser.

Bevorzugt ist die Federlasche an den Anbindungsabschnitt mit einem Linienkontakt angeformt/ angebunden. Der Begriff des Linienkontaktes ist dabei so zu verstehen, dass ein Querschnitt der Anformung/ Anbindung geradlinig ist. Die Federlasche ist auf diese Weise vergleichbar mit einem Scharnier an den Anbindungsabschnitt angebunden.

Um einen Bewegungsspielraum der Federlasche zum Lagerabschnitt sicherzustellen, ist gemäß einer Weiterbildung zwischen der Federlasche und dem Lagerabschnitt ein Schlitz oder Abstand vorgesehen.

Gemäß einer Weiterbildung, mit der ein Federweg des Grundkörpers relativ zur Federlasche beeinflussbar ist, erstreckt sich vom Grundkörper und/oder von der Federlasche weg wenigstens ein Schenkel, von dem eine Wegbegrenzung oder ein Anschlag bezüglich einem Einfedern und/oder Ausfedern der Federlasche gebildet ist, oder von dem eine Kraftabstützung gebildet ist.

Um eine Federkraft zu erhöhen oder wegabhängig besser einstellen zu können und/ oder um die Kraftbeaufschlagung des Klemmbackens besser steuern zu können, insbesondere in unterschiedlichen Bewegungs-, Kraft- und/ oder Einfeder-/ Ausfederrichtungen, sind gemäß einer Weiterbildung mehrere Federlaschen am Grundkörper angebunden, die sich insbesondere in unterschiedliche Bewegungs-, Kraft- und/ oder Einfeder-/ Ausfederrichtungen erstrecken.

Insbesondere ist wenigstens eine Federlasche vorgesehen, die eine Bewegung des beweglichen Klemmbackens in die Offenstellung unterstützt oder dämpft, und eine andere, die in Richtung der Klemmstellung des beweglichen Klemmbackens wirksam ist.

### Figurenbeschreibung

Die Offenbarung wird im Folgenden anhand von zwei beispielhaften, nicht einschränkenden, in den Figuren gezeigten Ausführungsformen näher erläutert. Dabei zeigt:
Figur 1 eine medizinische Schlauchpumpe gemäß einem Ausführungsbeispiel in einer Ansicht von vorne,
Figur 2 die Schlauchpumpe gemäß Figur 1 mit geöffneter Frontklappe,
Figur 3 einen Klemmmechanismus der medizinischen Schlauchpumpe gemäß Figur 1 und 2 in einer perspektivischen Ansicht,
Figur 4 einen Sperrhebel gemäß einem ersten Ausführungsbeispiel, der im Klemmmechanismus gemäß Figur 3 verbaut ist, in einer perspektivischen Ansicht,
Figur 5 den Sperrhebel gemäß Figur 4, in einer Ansicht von unten,
Figur 6 den Sperrhebel gemäß Figur 4 und 5, in einer Seitenansicht, und
Figur 7 einen Sperrhebel gemäß einem zweiten Ausführungsbeispiel, der im Klemmmechanismus gemäß Figur 3 verbaubar ist.

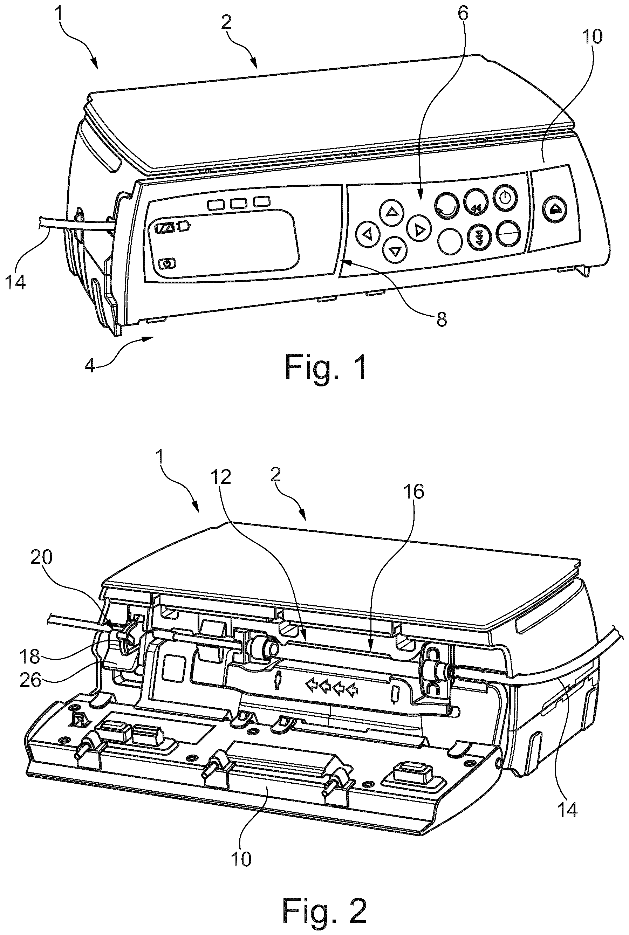

Figur 1 zeigt eine als Infusionsgerät ausgestaltete medizinische Schlauchpumpe 1 in einer perspektivischen Ansicht von vorne. Das Infusionsgerät, bzw. die Schlauchpumpe 1, hat ein im wesentlichen quaderförmiges Gehäuse 2 mit einer Frontseite 4, die von einem unterseitig anscharnierten, klappbaren Schlauchfachdeckel 10 gebildet ist, an dem ein Bedienfeld 6 und ein Display 8 angeordnet sind. Vom Schlauchfachdeckel 10 ist ein Schlauchfach 12 verschlossen, in den ein Infusionsschlauch 14 eingelegt wird (vgl. auch Figur 2).

Figur 2 zeigt das Infusionsgerät 1 gemäß Figur 1 in einer perspektivischen Ansicht von vorne mit geöffnetem Schlauchfach 12, das heißt mit aufgeklapptem Schlauchfachdeckel 10. Dabei erstreckt sich gemäß Figur 2 von rechts, ausgehend von einem Infusionsbehälter (nicht dargestellt), der Infusionsschlauch 14 in das Schlauchfach 12 hinein. Der Infusionsschlauch 14 ist bestimmungsgemäß eingelegt.

Das Schlauchfach 12 bildet zusammen mit dem Schlauchfachdeckel 10 eine offenbarungsgemäße Schlauchaufnahme, die ausgestaltet ist, dass in sie der Infusionsschlauch 14 einlegbar und - im gezeigten Ausführungsbeispiel durch Schließen des Schlauchfachdeckels 10 - festlegbar ist.

Das Schlauchfach 12 hat einen Schlaucheingang 20 im Bereich einer aus Bedienungssicht rechten Seitenwand, sowie einen Schlauchausgang im Bereich einer linken Seitenwand. Der gemäß Figur 2 niedergeklappte Schlauchfachdeckel 10 bildet zusammen mit einem Boden oder Grund des Schlauchfaches 12 Formabschnitte aus, in die der Infusionsschlauch 14 in seine Betriebslage eingelegt ist. Durch das Schließen des Schlauchfachdeckels 10 wird der Infusionsschlauch 14 in dieser Betriebslage fixiert.

Mit Bezug zur Förderrichtung von rechts nach links gemäß Figur 2, ist rechts am Grund des Schlauchfaches 12 eine im Wesentlichen rechteckige Aussparung 16 vorgesehen, die von einer Membran überspannt ist, hinter der lineare Verdränger angeordnet sind, die den Infusionsschlauch 14 im Betrieb in peristaltischer Abfolge gegen den geschlossenen Schlauchfachdeckel 10 quetschen und somit Fluid, von rechts nach links, hin zu dem Patienten fördern.

Um sicherzustellen, dass der Fluidfluss zum Patienten ausschließlich mittels der Schlauchpumpe 1 und nicht vorzeitig erfolgt, beispielsweise bereits beim Einlegen des Infusionsschlauches 14 in das Schlauchfach 12, ist ein redundantes Klemmensystem vorgesehen. Zum einen ist hierzu an dem Infusionsschlauch 14 selbst bereits eine Schlauchklemme 18 fest vorgesehen, zum anderen verfügt die Schlauchpumpe 1 im Gehäuse 2 über einen Klemmenmechanismus, der weitestgehend innerhalb des Gehäuses 2 angeordnet ist und daher in Figur 2 nicht dargestellt ist. Zur besseren Orientierung ist jedoch in Figur 2 eine Lage des Klemmenmechanismus mit dessen Bezugszeichen 20 angedeutet, woraus ersichtlich ist, dass er in enger räumlicher Nähe zur eingesetztem Schlauchklemme 18 angeordnet ist. Der Klemmenmechanismus 20 wird dabei beim Einlegen des Infusionsschlauches 14 durch das Einstecken der Schlauchklemme 18 in den Klemmenmechanismus 20 und das folgende Schließen des Schlauchfachdeckels 10 aktiviert.

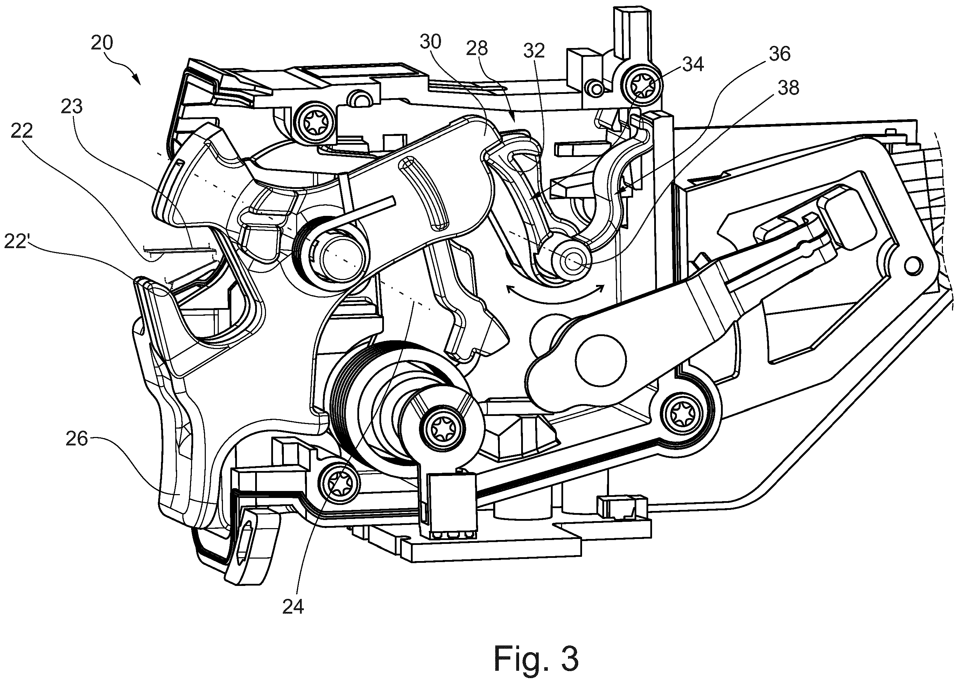

Figur 3 zeigt den freigestellten Klemmmechanismus 20 in einer Ansicht von der Seite, die gemäß Figur 2 etwa einer Blickrichtung von rechts nach links entspricht. Der Klemmmechanismus 20 hat zwei Klemmbacken 22 und 23, von denen in Figur 3 lediglich Klemmabschnitte schematisch und in Klemmstellung dargestellt sind. Die Klemmabschnitte weisen in der dargestellten Klemmstellung (22, 23) einen geringen, vorbestimmten Abstand auf, der etwa einem doppelten Wandungsdurchmesser des Infusionsschlauchs 14 entspricht, sodass dieser in der Klemmstellung vollständig geklemmt ist, jedoch nicht beschädigt wird. Der zweite Klemmbacken 23 ist fest.

Der erste Klemmbacken 22 ist um eine Rotationsachse 24 rotierbar, das heißt beweglich. Der erste Klemmbacken 22 ist mit einem, von einem Anwender tastbaren, Druckhebel 26 (vgl. auch Figur 2) gekoppelt, über den er in eine in Figur 3 dargestellte Offenstellung, repräsentiert durch das Bezugszeichen 22`, betätigt werden kann, in welcher der erste Klemmbacken 22' offensteht und mit dem zweiten Klemmbacken 23 eine maulartige Öffnung anbietet, in die der Infusionsschlauch 14 eingelegt werden kann. (vgl. Fig. 2, hier eingelegter Infusionsschlauch 14, Klemmstellung).

In der Offenstellung gemäß Figur 3 ist der in die Offenstellung betätigte, bewegliche Klemmbacken 22' offenbarungsgemäß mittelbar, im gezeigten Ausführungsbeispiel über den Druckhebel 26, mit einem Sperrhebel 28 des Klemmenmechanismus 20 verrastet, und auf diese Weise in seiner Offenstellung festgelegt. Die Verrastung ist realisiert, indem ein Rastnocken 30 des Druckhebels 26 auf einem Rastabschnitt 32 des Sperrhebels 28 aufsitzt.

Der Sperrhebel 28 ist offenbarungsgemäß von einem den Rastabschnitt 32 aufweisenden Grundkörper 34 und einer Federlasche 36 gebildet. Letztgenannte ist mit dem Grundkörper 34 offenbarungsgemäß einstückig ausgebildet. Hierzu ist die Federlasche 36 an den Grundkörper 34 angeformt. Die Federlasche 36 erstreckt sich von dem Grundkörper 34 S-förmig geschwungen. Der Sperrhebel 28 ist um eine Rotationsachse 38 drehbar gelagert und weist in einer Rotationsebene eine im wesentlichen V-förmige Grundform auf

Die Federlasche 36 ist mit einem vom Grundkörper 34 entfernten Endabschnitt gehäuseseitig abgestützt und weist eine Vorspannung auf. Auf diese Weise ist der Grundkörper 34 gegen den Druckhebel 26 gespannt, über den der erste Klemmbacken 22' mittelbar in seiner Offenstellung gesichert ist.

Mit dem Einlegen des Infusionsschlauches 14 in die Schlauchpumpe 1 wird die Schlauchklemme 18 in ihren zugehörigen Schacht im Klemmenmechanismus 20 eingeschoben (vgl. Fig. 2) und betätigt dabei im Inneren des Gehäuses 2 den Sperrhebel 28, indem sie dessen Grundkörper 34 mittelbar (über einen nicht dargestellten Intermediärhebel) und u.a. entgegen der Federkraft der Federlasche 36, um die Rotationsachse 38 im Uhrzeigersinn aus der in Figur 3 dargestellten Offenstellung/Verrastungsposition bewegt. Damit schnappt der Druckhebel 26 aufgrund seiner Vorspannung im Uhrzeigersinn um die Rotationsachse 24 und nimmt den ersten, beweglichen Klemmbacken 22`, ebenso im Uhrzeigersinn, in seine Klemmstellung (repräsentiert durch das Bezugszeichen 22) mit und der Klemmmechanismus 20 klemmt den Infusionsschlauch 14 stromabwärts der Schlauchklemme 18 ab (vgl. Position des Druckabschnitts 26, Fig. 2).

Mittels dem Schließen des Schlauchfachdeckels 10 schiebt dieser - im Anschluss an das zuvor beschriebene Schließen des Klemmmechanismus 20 - die als Schiebeklemme ausgestaltete Schlauchklemme 18 in ihre Offenstellung.

Auf diese Weise wurde die Klemmfunktion der Schlauchklemme 18 automatisiert - durch das Einlegen des Infusionsschlauches 14 und das Schließen des Schlauchfachdeckels 10 - an die Klemmfunktion des Klemmmechanismus übergeben. Der Fluidfluss erfolgt erst, wenn ein Anwender über das Bedienfeld 6 den entsprechenden Befehl eingibt.

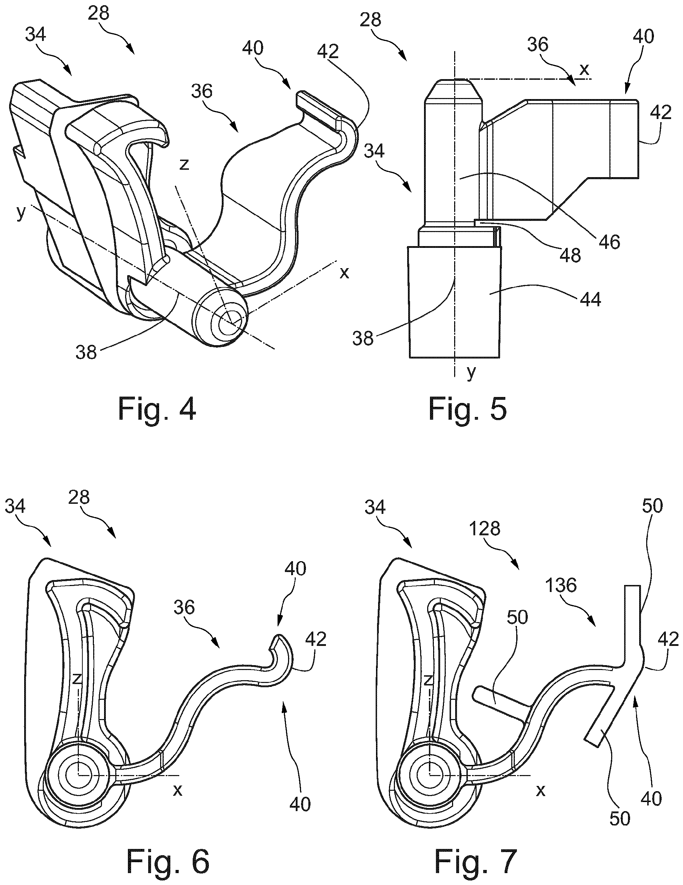

Die Figur 4 zeigt den Sperrhebel 28 in einer perspektivischen Ansicht gemäß Figur 3, in freigestellter Darstellung. Hierbei ist gut zu erkennen, dass ein Endabschnitt 40, mit dem die Federlasche 36 gehäuseseitig abgestützt ist (vgl. Figur 3), eine zur gehäuseseitigen Abstützfläche hin konvex gekrümmte, teilzylindrische Außenmantelfläche 42 hat mittels der er gut abgleiten kann. Um einen Bewegungsspielraum der Federlasche 36 zum Lagerabschnitt 44 sicherzustellen, ist gemäß Figur 4 zwischen der Federlasche 36 und dem Lagerabschnitt 44 ein Schlitz 48 vorgesehen.

Figur 5 zeigt den freigestellten Sperrhebel 28 gemäß Figur 4 in einer Ansicht von unten und verdeutlicht in Zusammenschau mit den Figuren 4 und 6, dass eine Breite der Federlasche 36 bemessen in einer Richtung senkrecht zur Rotationsebene, was in den Figuren die Richtung der Rotationsachse 38 ist (auch als y bezeichnet), etwa ein 5- bis 10-faches einer Dicke der Federlasche 36 beträgt, wobei die Dicke in der Rotationsebene bemessen ist, das heißt in der x-z Ebene gemäß Figur 4, bzw. Figur 6. Bevorzugt beträgt diese Breite wenigstens das 2-fache der Dicke der Federlasche 36. Auf diese Weise weist die Federlasche 36 eine hohe Torsionssteifigkeit auf, sodass insbesondere beim Einfedern ein seitliches Wegknicken und/ oder Ausweichen der Federlasche 36 verhindert ist.

Figur 6 zeigt den freigestellten Sperrhebel 28 gemäß Figur 4, bzw. 5 in einer Ansicht von der Seite, wobei insbesondere die S-förmig geschwungene Form der Federlasche 36 verdeutlicht wird.

Figur 7 zeigt einen Sperrhebel 128 gemäß einem zweiten Ausführungsbeispiel, der ebenso im Klemmmechanismus 20 gemäß Figur 3 verbaubar ist, in einer Ansicht von der Seite. Abweichend vom Ausführungsbeispiel gemäß den Figuren 3 bis 6 erstrecken sich hierbei mehrere Schenkel 50 weg von der Federlasche 36, wodurch eine Wegbegrenzung oder ein Anschlag bezüglich dem Einfedern der Federlasche 36 gegen den Grundkörper 34 und Ausfedern der Federlasche 36, sowie eine zusätzliche Kraftabstützung der Federlasche 36 in deren Endstellungen gebildet ist.

### Referenzzeichenliste

- 1: Schlauchpumpe
- 2: Gehäuse
- 4: Frontseite
- 6: Bedienfeld
- 8: Display
- 10: Schlauchfachdeckel
- 12: Schlauchfach
- 14: Infusionsschlauch
- 16: Aussparung
- 18: Schlauchklemme
- 20: Klemmmechanismus
- 22: erster Klemmbacken (Klemmstellung)
- 22': erster Klemmbacken (Offenstellung)
- 23: zweiter Klemmbacken
- 24: Rotationsachse
- 26: Druckhebel
- 28; 128: Sperrhebel
- 30: Rastnocken
- 32: Rastabschnitt
- 34: Grundkörper
- 36; 136: Federlasche
- 38: Rotationsachse
- 40: Endabschnitt
- 42: Außenmantelfläche
- 44: Lagerabschnitt
- 46: Anbindungsabschnitt
- 48: Schlitz
- 50: Schenkel

## Patentansprüche

1. Medizinische Schlauchpumpe (1), insbesondere zur medizinischen Dosierung, mit einer Schlauchaufnahme, die ausgestaltet ist, dass in sie ein Schlauch (14) einlegbar und festlegbar ist, und mit einem Klemmmechanismus (20) mit einem ersten Klemmbacken (22, 22`), der gegen einen zweiten Klemmbacken (23) in eine Klemmstellung vorgespannt ist, in welcher der Schlauch (14) klemmbar ist, insbesondere geklemmt ist, wobei der erste Klemmbacken (22, 22`) aus der Klemmstellung in eine Offenstellung betätigbar ist, in welcher der Schlauch (14) freigebbar, insbesondere freigegeben, und/oder einlegbar ist, und mit einem Sperrhebel (28), der von wenigstens einem Federelement (36) mittelbar oder unmittelbar gegen den ersten Klemmbacken (22, 22`) spannbar ist, insbesondere gespannt ist, sodass der erste Klemmbacken (22, 22`) in der Offenstellung mittelbar oder unmittelbar am Sperrhebel (28) abgestützt ist, **dadurch gekennzeichnet, dass** der Sperrhebel (28) mit dem Federelement (36) einstückig, insbesondere stoffeinstückig, gebildet ist.

2. Schlauchpumpe (1) nach Anspruch 1, **dadurch gekennzeichnet, dass** das Federelement als Federlasche (36) ausgebildet ist, die an einen Grundkörper (34) des Sperrhebels (28) angeformt ist.

3. Schlauchpumpe (1) nach Anspruch 2, **dadurch gekennzeichnet, dass** der Grundkörper (34) gehäuseseitig (2) rotierbar gelagert ist, wobei die Federlasche (36) in einer Rotationsebene gegen den Grundkörper (34) abgewinkelt ist, wobei die Federlasche (36) gehäuseseitig abgestützt ist und der Grundkörper (34) von der Federlasche (36) mittelbar oder unmittelbar gegen den ersten Klemmbacken (22, 22`) gespannt ist.

4. Schlauchpumpe (1) nach Anspruch 2 oder 3, **dadurch gekennzeichnet, dass** die Federlasche (36) geschwungen, insbesondere S-förmig geschwungen ausgebildet ist.

5. Schlauchpumpe (1) nach einem der vorhergehenden Ansprüche 2 bis 4, **dadurch gekennzeichnet, dass** eine Richtung, mit der die Federlasche (36) an den Grundkörper (34) angeformt, insbesondere angebunden, ist, eine Rotationsachse (38) des Sperrhebels (28) schneidet.

6. Schlauchpumpe (1) nach Anspruch 2 bis 5, **dadurch gekennzeichnet, dass** ein Endabschnitt (40), mit dem die Federlasche (36) gehäuseseitig abgestützt ist, eine zu einer gehäuseseitigen Abstützfläche hin konvex gekrümmte, insbesondere teilzylindrische, Außenmantelfläche (42) hat.

7. Schlauchpumpe (1) nach einem der Ansprüche 2 bis 6, **dadurch gekennzeichnet, dass** eine Breite der Federlasche (36), insbesondere bemessen in einer Richtung senkrecht zur Rotationsebene, insbesondere in Richtung der Rotationsachse (38), wenigstens ein 2- bis 10-faches einer Dicke der Federlasche (36), insbesondere bemessen in der Rotationsebene, beträgt

8. Schlauchpumpe (1) nach einem der Ansprüche 2 bis 7, **dadurch gekennzeichnet, dass**, der Grundkörper (34) einen Lagerabschnitt (44) hat, der gehäuseseitig rotierbar gelagert ist, und einen zu dem Lagerabschnitt (44) verjüngten Anbindungsabschnitt (46) hat, an dem die Federlasche (36) angebunden ist, wobei zwischen der Federlasche (36) und dem Lagerabschnitt (44) ein Schlitz (48) oder Abstand vorgesehen ist.

9. Schlauchpumpe (1) nach einem der Ansprüche 2 bis 8, **dadurch gekennzeichnet, dass** sich vom Grundkörper (34) und/oder von der Federlasche (36) wenigstens ein Schenkel (50) weg erstreckt, von dem eine Wegbegrenzung oder ein Anschlag bezüglich einem Einfedern und/oder Ausfedern der Federlasche (36) gebildet ist, und/oder von dem oder denen eine Kraftabstützung gebildet ist.

10. Schlauchpumpe nach einem der Ansprüche 2 bis 9, **dadurch gekennzeichnet, dass** mehrere Federlaschen am Grundkörper angebunden sind, insbesondere eine, die eine Bewegung des ersten Klemmbackens in die Offenstellung unterstützt oder dämpft, und eine andere, die in Richtung der Klemmstellung wirksam ist.
